# NEUE EUROPÄISCHE PATENTSCHRIFT

(11) **EP 2 276 449 B2**
(45) Veröffentlichungstag und Bekanntmachung des Hinweises auf die Entscheidung über den Einspruch: **29.12.2021**
(45) Hinweis auf die Patenterteilung: 21.09.2016
(21) Anmeldenummer: 09729866.5
(22) Anmeldetag: 12.03.2009
(51) Int. Cl.: A61K 8/33, A61K 8/34, A61K 8/37, A61K 8/41, A61Q 17/04, A61Q 19/00

(54) **PARFÜMIERTE KOSMETISCHE ZUBEREITUNG**
PERFUMED COSMETIC PREPARATION
PRÉPARATION COSMÉTIQUE PARFUMÉE

(30) Priorität: 11.04.2008 DE 102008018788
(43) Veröffentlichungstag der Anmeldung: 26.01.2011
(60) Teilanmeldung: 14174032.4
(73) Patentinhaber: Beiersdorf AG, 20253 Hamburg (DE)
(72) Erfinder: ESPEL, Anja, 22041 Hamburg (DE); BLOHM, Alexandra, 20257 Hamburg (DE); SKUBSCH, Kerstin, 25497 Prisdorf (DE); FEY, Sven, 22397 Hamburg (DE); RUPPERT, Stephan, 20259 Hamburg (DE)
(74) Vertreter: Beiersdorf AG
(86) Internationale Anmeldenummer: PCT/EP2009/001789
(87) Internationale Veröffentlichungsnummer: WO 2009/124632

(56) Entgegenhaltungen:
- EP-A1- 1 994 921
- EP-B1- 1 046 391
- DE-U1-202006 005 494
- JP-A- 63 048 209
- DATABASE GNPD [Online] MINTEL Juli 2006 'Advanced Anti-Aging System 45+' Database accession no. 538775
- DATABASE GNPD [Online] MINTEL April 2007 'Eau de Toilette' Database accession no. 698662
- DATABASE GNPD [Online] MINTEL August 2007 'Day Cream' Database accession no. 762716
- DATABASE GNPD [Online] MINTEL August 2006 'SPF 50+ Anti-Brown-Spot' Database accession no. 577840

## Beschreibung

Die vorliegende Erfindung betrifft eine parfümierte kosmetische Zubereitung.

Der Wunsch, schön und attraktiv auszusehen, ist von Natur aus im Menschen verwurzelt. Auch wenn das Schönheitsideal im Laufe der Zeit Wandlungen erfahren hat, so ist das Streben nach einem makellosen Äußeren immer das Ziel der Menschen gewesen. Einen wesentlichen Anteil an einem schönen und attraktiven Äußeren hat dabei der Zustand und das Aussehen der Haut.

Damit die Haut ihre biologischen Funktionen im vollen Umfang erfüllen kann, bedarf sie der regelmäßigen Reinigung und Pflege. Die Reinigung der Haut dient dabei der Entfernung von Schmutz, Schweiß und Resten abgestorbener Hautpartikel, die einen idealen Nährboden für Krankheitserreger und Parasiten aller Art bilden. Hautpflegeprodukte dienen meist der Befeuchtung und Rückfettung der Haut. Häufig sind ihnen Wirkstoffe zugesetzt, welche die Haut regenerieren oder vor den schädlichen Einflüssen der UV-Strahlung schützen.

Kosmetika enthalten normalerweise eine Reihe von Parfümstoffen, die dazu dienen sollen, unangenehme Eigengerüche von Zubereitungsbestandteilen zu überdecken und dem Kosmetikum den individuellen, Hersteller-typischen Geruch zu verleihen. Nachteilig am Stande der Technik ist jedoch, dass einige kosmetische Inhaltsstoffe wie 2-(4'-Diethylamino-2'-hydoxybenzoyl)-benzoesäurehexylester dazu führen, dass die kosmetischen Zubereitungen vorzeitig oder beschleunigt ihren Duft verlieren oder die Zubereitung, beispielsweise durch vorzeitigen Abbau einiger Parfümbestandteile, sich in ihrem Duft ändert.

Es war daher die Aufgabe der vorliegenden Erfindung, die Mängel des Standes der Technik zu beseitigen und eine kosmetische Zubereitung zu entwickeln, die trotz eines Gehaltes an 2-(4'-Diethylamino-2'-hydoxybenzoyl)-benzoesäurehexylester geruchsstabil ist.

Es war darüber hinaus die Aufgabe der vorliegenden Erfindung, eine parfümierte und geruchsstabile kosmetische Sonnenschutzformulierung zu entwickeln, die sich insbesondere durch einen hohen UVA-Schutz und/oder eine ausgewogene UVA-Balance auszeichnet. Die Zubereitung sollte einfach und kostengünstig herstellbar sein.
durch einen hohen UVA-Schutz und/oder eine ausgewogene UVA-Balance auszeichnet. Die Zubereitung sollte einfach und kostengünstig herstellbar sein.

Überraschend gelöst werden die Aufgaben durch eine kosmetische Zubereitung in Form einer Emulsion oder Dispersion gemäß Anspruch 1.

Überraschend gelöst werden die Aufgaben durch die Verwendung von 2-(4'-Diethylamino-2'-hydoxybenzoyl)-benzoesäurehexylester zur Stabilisierung des Geruches von kosmetischen Zubereitungen enthaltend eine oder mehrere Parfümstoffe gewählt aus der Gruppe der Verbindungen Linalool, Hexylcinnamal, Benzylsalicylat.

Im Rahmen dieser Offenbarung beziehen sich die Formulierungen "erfindungsgemäß vorteilhaft", "erfindungsgemäß bevorzugt" etc. sowohl auf die erfindungsgemäße Zubereitung.

Zwar kennt der Stand der Technik die EP1994921, JP63048209, DE202006005494 sowie die in der GMPD Datenbank (MINTEL) offenbarten Zubereitungen "Advanced Anti-Aging System 45+" (Datenbankeintrag Nr. 538775), "Eau de Toilette" (Datenbank Nr. 698662) und "Day Cream" (Datenbank Nr. 762716), doch konnten diese Zubereitungen nicht den Weg zur vorliegenden Erfindung weisen.

Es ist erfindungsgemäß bevorzugt, wenn die Gesamtkonzentration an Parfümstoffen in der Zubereitung von 0,00005 bis 0,5 Gew.-%, bezogen auf das Gesamtgewicht der Zubereitung, beträgt.

Es ist erfindungsgemäß vorteilhaft, wenn die Konzentration an Linalool in der Zubereitung von 0,00001 bis 0,1 Gew.-%, bezogen auf das Gesamtgewicht der Zubereitung, beträgt.

Es ist erfindungsgemäß bevorzugt, wenn die Konzentration an Linalool in der Zubereitung von 0,00005 bis 0,05 Gew.-%, bezogen auf das Gesamtgewicht der Zubereitung, beträgt.

Es ist erfindungsgemäß vorteilhaft, wenn die Konzentration an Hexylcinnamal in der Zubereitung von 0,00001 bis 0,1 Gew.-%, bezogen auf das Gesamtgewicht der Zubereitung, beträgt.

Es ist erfindungsgemäß bevorzugt, wenn die Konzentration an Hexylcinnamal in der Zubereitung von 0,00005 bis 0,05 Gew.-%, bezogen auf das Gesamtgewicht der Zubereitung, beträgt.

Es ist erfindungsgemäß vorteilhaft, wenn die Konzentration an Benzylsalicylat in der Zubereitung von 0,00001 bis 0,1 Gew.-%, bezogen auf das Gesamtgewicht der Zubereitung, beträgt.

Es ist erfindungsgemäß bevorzugt, wenn die Konzentration an Benzylsalicylat in der Zubereitung von 0,00005 bis 0,05 Gew.-%, bezogen auf das Gesamtgewicht der Zubereitung, beträgt.

Erfindungsgemäß vorteilhafte Ausführungsformen der vorliegenden Erfindung sind dadurch gekennzeichnet, dass die Zubereitung 2-(4'-Diethylamino-2'-hydoxybenzoyl)-benzoesäurehexylester in einer Konzentration von 0,1 bis 10 Gew.-%, bezogen auf das Gesamtgewicht der Zubereitung, beträgt.

Erfindungsgemäß bevorzugte Ausführungsformen der vorliegenden Erfindung sind dadurch gekennzeichnet, dass die Zubereitung 2-(4'-Diethylamino-2'-hydoxybenzoyl)-benzoesäurehexylester in einer Konzentration von 0,5 bis 8 Gew.-%, bezogen auf das Gesamtgewicht der Zubereitung, beträgt.

Es ist erfindungsgemäß vorteilhaft, wenn die Zubereitung als weitere Komponenten eine oder mehrere Verbindungen gewählt aus der Gruppe der Verbindungen Limonen, Citral, alpha-Isomethylionon, Geraniol, Methyl-2-Octynoat, Citronellol, Isoeugenol, 2-Isobutyl-4-hydroxy-4-methyltetrahydropyran, 2-tert-Pentylcyclohexylacetat, 3-Methyl-5-phenyl-1-pentanol, 7-Acetyl-1,1,3,4,4,6-hexamethyltetralin, Adipinsäurediester, alpha-Amylcinnamaldehyd, Alpha-Methylionon, Amyl C Butylphenylmethylpropionalcinnamal, Amylsalicylat, Amylcinnamylalkohol, Anisalkohol, Benzoin, Benzylalkohol, Benzylbenzoat, Benzylcinnamat, Bergamotöl, bitteres Orangenöl, Butylphenylmethylpropioal, Cardamomöl, Cedrol, Cinnamal, Cinnamylalkohol, Citronellylmethylcrotonat, Citronenöl, Coumarin, Diethylsuccinat, d-Limonene, Ethyllinalool, Eugenol, Evernia Furfuracea Extract, Evernia Prunastri Extract, Farnesol, Guajakholzöl, Hexylsalicylat, Hydroxycitronellal, Hydroxyisohexyl 3-Cyclohexencarboxaldehyde, Lavendelöl, Lemonenöl, Linaylacetat, Mandarinenöl, Menthyl PCA, Methylheptenon, Muskatnussöl, Rosmarinöl, süßes Orangenöl, Terpineol, Tonkabohnenöl, Triethylcitrat und/oder Vanillin enthält.

Es ist erfindungsgemäß besonders bevorzugt, wenn die erfindungsgemäße Zubereitung in Form einer O/W-Emulsionen vorliegt.

Liegt die erfindungsgemäße Zubereitung in Form einer O/W-Emulsion vor, so ist sie erfindungsgemäß bevorzugt dadurch gekennzeichnet, dass die Zubereitung einen oder mehrere O/W-Emulgatoren gewählt aus der Gruppe der Verbindungen Glycerylstearatcitrat, Glycerylstearat (selbstemulgierend), Stearinsäure, Stearatsalze, Polyglyceryl-3-methylglycosedistearat, Ceteareth-20, PEG-40 Stearat, Natriumcetearylsulfat, Sucrose Polystearat, Natrium Stearoyl Glutamat enthält. Ferner ist es vorteilhaft im Sinne der vorliegenden Erfindung Cetearylalkohol in Kombination mit PEG-40 hydriertes Rizinusöl, Natriumcetearylsulfat und Glycerylstearat. Außerdem ist es erfindungsgemäß vorteilhaft Kaliumcetylphosphat als Emulgator einzusetzen.

Diese erfindungsgemäßen O/W-Emulgatoren können erfindungsgemäß vorteilhaft in einer Konzentration von 0,001 bis 10 Gewichts-% und bevorzugt in einer Konzentration von 0,1 bis 7 Gewichts-%, bezogen auf das Gesamtgewicht der Zubereitung in dieser enthalten sein.

In einer weiteren erfindungsgemäßen Ausführungsform liegt die erfindungsgemäße Zubereitung in Form einer W/O-Emulsion vor.

In dieser Ausführungsform ist es erfindungsgemäß bevorzugt, wenn die Zubereitung einen oder mehrere W/O-Emulgatoren gewählt aus der Gruppe der Verbindungen Polyglyceryl-2-dipolyhydroxystearat, PEG-30 Dipolyhydroxystearat, Cetyl Dimethicon Copolyol, Polyglyceryl-3 Diisostearat enthält.

Diese erfindungsgemäßen W/O-Emulgatoren können erfindungsgemäß vorteilhaft in einer Konzentration von 0,1 bis 10 Gewichts-% und bevorzugt in einer Konzentration von 0,2 bis 7 Gewichts-%, bezogen auf das Gesamtgewicht der Zubereitung in dieser enthalten sein.

Es ist erfindungsgemäß vorteilhaft, wenn die erfindungsgemäße Zubereitung frei ist von p-Methylbenzylidencampher.

Es ist dabei erfindungsgemäß vorteilhaft, wenn die erfindungsgemäße Zubereitung einen oder mehrere dieser zusätzlichen UV-Filter in einer Gesamtkonzentration von 0,1 bis 40 Gewichts-% und bevorzugt in einer Konzentration von 1 bis 30 Gewichts-%, bezogen auf das Gesamtgewicht der Zubereitung, enthält.

Die Pigmente (Titandioxid, Zinkoxid) können vorteilhaft im Sinne der vorliegenden Erfindung auch in Form kommerziell erhältlicher öliger oder wässriger Vordispersionen zur Anwendung kommen. Diesen Vordispersionen können vorteilhaft Dispergierhilfsmittel und/oder Solubilisationsvermittler zugesetzt sein.

Die Pigmente (Titandioxid, Zinkoxid) können erfindungsgemäß vorteilhaft oberflächlich behandelt ("gecoatet") sein, wobei beispielsweise ein hydrophiler, amphiphiler oder hydrophober Charakter gebildet werden bzw. erhalten bleiben soll. Diese Oberflächenbehandlung kann darin bestehen, dass die Pigmente nach an sich bekannten Verfahren mit einer dünnen hydrophilen und/oder hydrophoben anorganischen und/oder organischen Schicht versehen werden. Die verschiedenen Oberflächenbeschichtungen können im Sinne der vorliegenden Erfindung auch Wasser enthalten.

Anorganische Oberflächenbeschichtungen im Sinne der vorliegenden Erfindung können bestehen aus Aluminiumoxid (Al₂O₃), Aluminiumhydroxid Al(OH)₃, bzw. Aluminiumoxidhydrat (auch: Alumina, CAS-Nr.: 1333-84-2), Natriumhexametaphosphat (NaPO₃)₆, Natriummetaphosphat (NaPO₃)ₙ, Siliciumdioxid (SiO₂) (auch: Silica, CAS-Nr.: 7631-86-9), Bariumsulfat (BaSO₄) oder Eisenoxid (Fe₂O₃). Diese anorganischen Oberflächenbeschichtungen können allein, in Kombination und/oder in Kombination mit organischen Beschichtungsmaterialien vorkommen.

Organische Oberflächenbeschichtungen im Sinne der vorliegenden Erfindung können bestehen aus pflanzlichem oder tierischem Aluminiumstearat, pflanzlicher oder tierischer Stearinsäure, Laurinsäure, Dimethylpolysiloxan (auch: Dimethicone), Methylpolysiloxan (Methicone), Simethicone (einem Gemisch aus Dimethylpolysiloxan mit einer durchschnittlichen Kettenlänge von 200 bis 350 Dimethylsiloxan-Einheiten und Silicagel) oder Alginsäure. Diese organischen Oberflächenbeschichtungen können allein, in Kombination und/oder in Kombination mit anorganischen Beschichtungsmaterialien vorkommen.

Es ist erfindungsgemäß vorteilhaft, wenn die Zubereitung als weitere Inhaltsstoffe eine oder mehrere Verbindungen gewählt aus der Gruppe der Verbindungen alpha-Liponsäure, Folsäure, Phytoen, D-Biotin, Coenzym Q10, Niacinamid, alpha-Glucosylrutin, Carnitin, Carnosin, natürliche und/oder synthetische Isoflavonoide, Flavonoide, Kreatin, Kreatinin, Taurin, ß-Alanin, Tocopherylacetat, Dihydroxyaceton; 8-Hexadecen-1,16-dicarbonsäure, Glycerylglycose, (2-Hydroxyethyl)harnstoff, Vitamin E bzw. seine Derivate und/oder Licochalcon A in den üblichen Einsatzkonzentrationen enthält.

Es ist erfindungsgemäß von Vorteil, wenn die Zubereitung ein oder mehrere Diole gewählt aus der Gruppe der Verbindungen 2-Methyl-1,3-propandiol, Propan-1,2-diol, Butan-1,2-diol, Pentan-1,2-diol, Hexan-1,2-diol, Heptan-1,2-diol, Octan-1,2-diol, Nonan-1,2-diol, Decan-1,2-diol enthält.

Derartige Diole können erfindungsgemäß vorteilhaft in einer Konzentration von 0,1 bis 20 Gewichts-%, bezogen auf das Gesamtgewicht der Zubereitung, in dieser enthalten sein.

Derartige Diole können erfindungsgemäß bevorzugt in einer Konzentration von 1 bis 10 Gewichts-%, bezogen auf das Gesamtgewicht der Zubereitung, in dieser enthalten sein.

Es ist erfindungsgemäß vorteilhaft, wenn die Zubereitung ein oder mehrere Parabene (z.B. Methylparaben, Ethylparaben, Propylparaben, Butylparaben) enthält. Dabei ist ein Gesamt-Paraben-Gehalt von 0,05 bis 1 Gewichts-%, bezogen auf das Gesamtgewicht der Zubereitung erfindungsgemäß vorteilhaft.

Die erfindungsgemäßen Zubereitungen können ferner vorteilhaft auch Selbstbräunungssubstanzen enthalten, wie beispielsweise Dihydroxyaceton und/oder Melaninderivate in Konzentrationen von 1 Gew.-% bis zu 10 Gew.-%, bezogen auf das Gesamtgewicht der Zubereitung.

Ferner können die erfindungsgemäßen Zubereitungen auch Repellentien zum Schutz vor Mücken, Zecken und Spinnen und dergleichen enthalten. Vorteilhaft sind z. B. N,N-Diethyl-3-methylbenzamid (Handelsbezeichnung: Meta-delphene, "DEET"), Dimethylphtalat (Handelsbezeichnung: Palatinol M, DMP), 1-Piperidincarbonsäure-2-(2-hydroxyethyl)-1-methylpropylester sowie insbesondere 3-(N-n-Butyl-N-acetyl-amino)-propionsäureethylester (unter dem Handelsnamen Insekt Repellent^{®} 3535 bei der Fa. Merck erhältlich). Die Repellentien können sowohl einzeln als auch in Kombination eingesetzt werden.

Als Feuchthaltemittel (Moisturizer) werden Stoffe oder Stoffgemische bezeichnet, welche kosmetischen Zubereitungen die Eigenschaft verleihen, nach dem Auftragen bzw. Verteilen auf der Hautoberfläche die Feuchtigkeitsabgabe der Hornschicht (auch transepidermal water loss (TEWL) genannt) zu reduzieren und/oder die Hydratation der Hornschicht positiv zu beeinflussen.

Vorteilhafte Feuchthaltemittel (Moisturizer) im Sinne der vorliegenden Erfindung sind beispielsweise Glycerin, Milchsäure und/oder Lactate, insbesondere Natriumlactat, Butylenglykol, Propylenglykol, Biosaccaride Gum-1, Glycine Soja, Ethylhexyloxyglycerin, Pyrrolidoncarbonsäure und Harnstoff. Ferner ist es insbesondere von Vorteil, polymere Moisturizer aus der Gruppe der wasserlöslichen und/oder in Wasser quellbaren und/oder mit Hilfe von Wasser gelierbaren Polysaccharide zu verwenden. Insbesondere vorteilhaft sind beispielsweise Hyaluronsäure, Chitosan und/oder ein fucosereiches Polysaccharid, welches in den Chemical Abstracts unter der Registraturnummer 178463-23-5 abgelegt und z. B. unter der Bezeichnung Fucogel^{®}1000 von der Gesellschaft SOLABIA S.A. erhältlich ist. Moisturizer können vorteilhaft auch als Antifaltenwirkstoffe zum Schutz vor Hautveränderungen, wie sie z. B. bei der Hautalterung auftreten, verwendet werden.

Es ist vorteilhaft im Sinne der vorliegenden Erfindung, wenn die erfindungsgemäße Zubereitung ein oder mehrere Feuchthaltemittel in einer Gesamtkonzentration von 0,1 bis 20 Gewichts-% und bevorzugt in einer Gesamtkonzentration von 0,5 bis 10 Gewichts-%, jeweils bezogen auf das Gesamtgewicht der Zubereitung, enthält.

Erfindungsgemäß vorteilhaft ist die erfindungsgemäße Zubereitung frei von Glucosylglyceriden (Glycerylglucose).

Die erfindungsgemäßen kosmetischen Zubereitungen können ferner vorteilhaft, wenngleich nicht zwingend, Füllstoffe enthalten, welche z. B. die sensorischen und kosmetischen Eigenschaften der Formulierungen weiter verbessern und beispielsweise ein samtiges oder seidiges Hautgefühl hervorrufen oder verstärken. Vorteilhafte Füllstoffe im Sinne der vorliegenden Erfindung sind Stärke und Stärkederivate (wie z. B. Tapiocastärke, Distärkephosphat, Aluminium- bzw. Natrium-Stärke Octenylsuccinat und dergleichen), Pigmente, die weder hauptsächlich UV-Filter- noch färbende Wirkung haben (wie z. B. Bornitrid etc.) und/oder Aerosile^{®} (CAS-Nr. 7631-86-9) und /oder Talkum.

Die Wasserphase der erfindungsgemäßen Zubereitungen kann vorteilhaft übliche kosmetische Hilfsstoffe enthalten, wie beispielsweise Alkohole, insbesondere solche niedriger C-Zahl, vorzugsweise Ethanol und/oder Isopropanol oder Polyole niedriger C-Zahl sowie deren Ether, vorzugsweise Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, Polymere, Schaumstabilisatoren, Elektrolyte, Selbstbräuner sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z. B. Hyaluronsäure, Xanthangummi, Hydroxypropylmethylcellulose, Copolymer aus Vinylpyrrolidon und Acrylsäure, besonders vorteilhaft aus der Gruppe der Polyacrylate, bevorzugt ein Polyacrylat aus der Gruppe der so genannten Carbopole, beispielsweise Carbopole der Typen 980, 981, 1382, 2984, 5984, jeweils einzeln oder in Kombination. Weitere erfindungsgemäß vorteilhafte Verdicker sind solche mit der INCI-Bezeichnung Acrylates/C10-30 Alkyl Acrylate Crosspolymer (z. B. Pemulen TR 1, Pemulen TR 2, Carbopol 1328 von der Fa. NOVEON) sowie Aristoflex AVC (INCI: Ammonium Acryloyldimethyltaurate/VP Copolymer).

Erfindungsgemäß vorteilhaft enthält die erfindungsgemäße Zubereitung Filmbildner. Filmbildner im Sinne der vorliegenden Erfindung sind Stoffe unterschiedlicher Zusammensetzung, die durch die folgende Eigenschaft charakterisiert sind: Löst man einen Filmbildner in Wasser oder anderen geeigneten Lösungsmitteln und trägt die Lösung dann auf die Haut auf, so bildet er nach dem Verdunsten des Lösemittels einen Film aus, der im wesentlichen dazu dient, die Lichtfilter auf der Haut zu fixieren und so die Wasserfestigkeit des Produktes zu steigern. Es ist insbesondere von Vorteil, die Filmbildner aus der Gruppe der Polymere auf Basis von Polyvinylpyrrolidon (PVP) 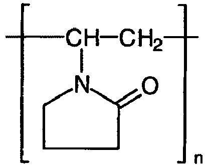 zu wählen. Besonders bevorzugt sind Copolymere des Polyvinylpyrrolidons, beispielsweise das PVP Hexadecen Copolymer und das PVP Eicosen Copolymer, welche unter den Handelsbezeichnungen Antaron V216 und Antaron V220 bei der GAF Chemicals Cooperation erhältlich sind.

Ebenfalls vorteilhaft sind weitere polymere Filmbildner, wie beispielsweise Natriumpolystryrensulfonat, welches unter der Handelsbezeichnung Flexan 130 bei der National Starch and Chemical Corp. erhältlich ist, und/oder Polyisobuten, erhältlich bei Rewo unter der Handelsbezeichnung Rewopal PIB1000. Weitere geeignete Polymere sind z.B. Polyacrylamide (Seppigel 305), Polyvinylalkohole, PVP, PVP / VA Copolymere, Polyglycole, Acrylat/Octylacralymid Copolymer (Dermacryl 79). Ebenfalls vorteilhaft ist die Verwendung von Hydriertem Rizinusöl Dimerdilinoleat (CAS 646054-62-8, INCl Hydrogenated Castor Oil Dimer Dilinoleate), das bei der Firma Kokyu Alcohol Kogyo unter dem Namen Risocast DA-H erworben werden kann oder aber auch PPG-3 Benzylethermyristat (CAS 403517-45-3), das unter dem Handelsnamen Crodamol STS bei der Firma Croda Chemicals erworben werden kann.

Die Ölphase der erfindungsgemäßen Zubereitung wird vorteilhaft gewählt aus der Gruppe der polaren Öle, beispielsweise aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z. B. Cocoglycerid, Olivenöl, Sonnenblumenöl, Jojobaöl, Sojaöl, Erdnußöl, Rapsöl, Mandelöl, Palmöl, Kokosöl, Rizinusöl, Weizenkeimöl, Traubenkernöl, Distelöl, Nachtkerzenöl, Macadamianußöl, Avocadoöl, Babassuöl und dergleichen mehr.

Erfindungsgemäß vorteilhaft sind ferner z. B. natürliche Wachse tierischen und pflanzlichen Ursprungs, wie beispielsweise Bienenwachs und andere Insektenwachse sowie Beerenwachs, Sheabutter und/oder Lanolin (Wollwachs), und/oder Karietéwachs.

Weitere vorteilhafte polare Ölkomponenten können im Sinne der vorliegenden Erfindung ferner gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Phenylethylbenzoat, 2-Phenylethylbenzoat, Isopropyl Lauroyl Sarkosinat, Phenyl Trimethicon, Cyclomethicon, Dibutyladipat, Octylpalmitat, Octylcocoat, Isopropyl palmitat, Isostearyl Isostearat, Cetearyl Isononanoate, Isopropyl Stearat, Octylisostearat, Octyldodeceylmyristat, Octyldodekanol, Cetearylisononanoat, Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Stearylheptanoat, Oleyloleat, Oleylerucat, Erucyloleat, Erucylerucat, Tridecylstearat, Tridecyltrimellitat, sowie synthetische, halbsynthetische und natürliche Gemische solcher Ester, wie z. B. Jojobaöl.

Ferner kann die Ölphase vorteilhaft gewählt werden aus der Gruppe der Dialkylether und Dialkylcarbonate, vorteilhaft sind z. B. Dicaprylylether (*Cetiol OE*) und/oder Dicaprylylcarbonat, beispielsweise das unter der Handelsbezeichnung *Cetiol CC* bei der Fa. Cognis erhältliche.

Es ist ferner bevorzugt, das oder die Ölkomponenten aus der Gruppe Isoeikosan, Neopentylglykoldiheptanoat, Propylenglykoldicaprylat/dicaprat, Caprylic/Capric/Diglycerylsuccinat, Butylenglykol Dicaprylat/Dicaprat, C₁₂₋₁₃-Alkyllactat, Di-C₁₂₋₁₃-Alkyltartrat, Triisostearin, Dipentaerythrityl Hexacaprylat/Hexacaprat, Propylenglykolmonoisostearat, Tricaprylin, Dimethylisosorbid. Es ist insbesondere vorteilhaft, wenn die Ölphase der erfindungsgemäßen Formulierungen einen Gehalt an C₁₂₋₁₅-Alkylbenzoat aufweist oder vollständig aus diesem besteht.

Vorteilhafte Ölkomponenten sind ferner z. B. Butyloctylsalicylat (beispielsweise das unter der Handelsbezeichnung *Hallbrite BHB* bei der Fa. CP Hall erhältliche), Tridecylsalicylat (welches unter der Handelsbezeichnung Cosmacol ESI bei der Fa. Sasol erhältlich ist), C12-C15 Alkylsalicylat (unter der Handelsbezeichnung Dermol NS bei der Fa. Alzo erhältlich), Hexadecylbenzoat und Butyloctylbenzoat und Gemische davon (*Hallstar AB*) und/oder Diethylhexylnaphthalat (*Hallbrite TQ oder Corapan TQ von Symrise)* sowie Propylheptyl Octanoat und/oder Diisopropylsebacat.

Auch beliebige Abmischungen solcher Öl- und Wachskomponenten sind vorteilhaft im Sinne der vorliegenden Erfindung einzusetzen.

Ferner kann die Ölphase ebenfalls vorteilhaft auch unpolare Öle enthalten, beispielsweise solche, welche gewählt werden aus der Gruppe der verzweigten und unverzweigten Kohlenwasserstoffe und -wachse, insbesondere Mineralöl, Vaseline (Petrolatum), Paraffinöl, Squalan und Squalen, Polyolefine, hydrogenierte Polyisobutene, C13-16 Isoparaffin und Isohexadecan. Unter den Polyolefinen sind Polydecene die bevorzugten Substanzen.

Die erfindungsgemäßen Zubereitungen können ferner vorteilhaft eine oder mehrere Substanzen aus der folgenden Gruppe der Siloxanelastomere enthalten, beispielsweise um die Wasserfestigkeit und/oder den Lichtschutzfaktor der Produkte zu steigern:
(a) Siloxanelastomere, welche die Einheiten R₂SiO und RSiO_{1,5} und/oder FbSiO_{0,5} und/oder SiO₂ enthalten,
   wobei die einzelnen Reste R jeweils unabhängig voneinander Wasserstoff, C₁₋₂₄-Alkyl (wie beispielsweise Methyl, Ethyl, Propyl) oder Aryl (wie beispielsweise Phenyl oder Tolyl), Alkenyl (wie beispielsweise Vinyl) bedeuten und das Gewichtsverhältnis der Einheiten R₂SiO zu RSiO_{1,5} aus dem Bereich von 1 : 1 bis 30 : 1 gewählt wird;
(b) Siloxanelastomere, welche in Silikonöl unlöslich und quellfähig sind, die durch die Additionsreaktion eines Organopolysiloxans (1), das siliciumbebundenen Wasserstoff enthält, mit einem Organopolysiloxan (2), das ungesättigte aliphatische Gruppen enthält, erhältlich sind,
   wobei die verwendeten Mengenateile so gewählt werden, dass die Menge des Wasserstoffes des Organopolysiloxans (1) oder der ungesättigten aliphatischen Gruppen des Organopolysiloxans (2)
   - im Bereich von 1 bis 20 mol-% liegt, wenn das Organopolysiloxan nicht zyklisch ist und
   - im Bereich von 1 bis 50 mol-% liegt, wenn das Organopolysiloxan zyklisch ist.

Vorteilhaft im Sinne der vorliegenden Erfindung liegen das oder die Siloxanelastomere in Form sphärischer Puder oder in Form von Gelen vor.

Erfindungsgemäß vorteilhafte in Form sphärischer Puder vorliegende Siloxanelastomere sind die mit der INCl-Bezeichnung Dimethicone / Vinyl Dimethicone Crosspolymer, beispielsweise das von DOW CORNING unter der Handelsbezeichnungen DOW CORNING 9506 Powder erhältliche.

Besonders bevorzugt ist es, wenn das Siloxanelastomer in Kombination mit Ölen aus Kohlenwasserstoffen tierischer und/oder pflanzlicher Herkunft, synthetischen Ölen, synthetischen Estern, synthetischen Ethern oder deren Gemischen verwendet wird.

Besonders vorteilhafte Zubereitungen werden ferner erhalten, wenn als Zusatz- oder Wirkstoffe Antioxidantien eingesetzt werden. Erfindungsgemäß enthalten die Zubereitungen vorteilhaft eines oder mehrere Antioxidantien. Als günstige, aber dennoch fakultativ zu verwendende Antioxidantien können alle für kosmetische Anwendungen geeigneten oder gebräuchlichen Antioxidantien verwendet werden.

Besonders vorteilhaft im Sinne der vorliegenden Erfindung können wasserlösliche Antioxidantien eingesetzt werden, wie beispielsweise Vitamine, z. B. Ascorbinsäure und deren Derivate.

Bevorzugte Antioxidantien sind ferner Vitamin E und dessen Derivate sowie Vitamin A und dessen Derivate.

Die Menge der Antioxidantien (eine oder mehrere Verbindungen) in den Zubereitungen beträgt vorzugsweise 0,001 bis 30 Gew.-%, besonders bevorzugt 0,05 bis 20 Gew.-%, insbesondere 0,1 bis 10 Gew.-%, bezogen auf das Gesamtgewicht der Zubereitung.

Die kosmetischen Zubereitungen gemäß der Erfindung können kosmetische Hilfsstoffe enthalten, wie sie üblicherweise in solchen Zubereitungen verwendet werden, z. B. Konservierungsmittel, Konservierungshelfer, Komplexbildner, Bakterizide, Parfüme, Substanzen zum Verhindern oder Steigern des Schäumens, Farbstoffe, Pigmente, die eine färbende Wirkung haben, Verdickungsmittel, anfeuchtende und/oder feuchthaltende Substanzen, Füllstoffe, die das Hautgefühl verbessern, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Vorteilhaft im Sinne der vorliegenden Erfindung sind Zubereitungen zur Pflege der Haut: sie können dem kosmetischen Lichtschutz, ferner als Schminkprodukt in der dekorativen Kosmetik dienen.

Entsprechend ihrem Aufbau können kosmetische Zusammensetzungen im Sinne der vorliegenden Erfindung, beispielsweise verwendet werden als Hautschutzcreme, Tages- oder Nachtcrème usw. Es ist gegebenenfalls möglich und vorteilhaft, die erfindungsgemäßen Zusammensetzungen als Grundlage für pharmazeutische Formulierungen zu verwenden.

Erfindungsgemäß ist insbesondere die Verwendung der erfindungsgemäßen Zubereitung zum Schutz vor Hautalterung (insbesondere zum Schutz vor UV-bedingter Hautalterung) sowie als Sonnenschutzmittel.

Erfindungsgemäß vorteilhaft weist die erfindungsgemäße Zubereitung einen pH-Wert von 5 bis 8 auf. Dieser kann durch die herkömmlichen Säuren, Basen und Puffersysteme eingestellt werden.
Erfindungsgemäß ist die Verwendung der Zubereitung in kosmetischen Sprays (insbesondere Sonnenschutzmitteln).

### Vergleichsversuche

Mit den folgenden Vergleichsversuchen konnte der erfinderische Effekt gezeigt werden:

### Durchführung

Parfümprüfungen stellen Rahmenbedingungen für einen Launch neuer Produkte dar, um die Qualität unserer Produkte zu sichern. Ein Bestandteil eines qualitativ hochwertigen Produktes ist die Stabilität der Parfümierung. Um diese Stabilität zu gewährleisten werden die entsprechenden Muster über einen Zeitraum von 6 Monaten einlagert.

| | |
|---|---|
| Die Lagerbedingungen sind: | + 6 ° C |
| | Raumtemperatur (RT) |
| | Brutschrank 40°C |
| | Licht |

So simulieren wir die bisher garantierten 3 Jahre Haltbarkeit unserer Produkte. Beachtet werden sollten der Füllgrad, die Siegelung, gleiche Verpackungsgröße bzw. Verpackungsart.

Die Muster werden nach 2, 4 und 6 Monaten Lagerung von Parfümevaluatoren olfaktorisch beurteilt. Dazu werden die Muster auf neutrale Pappkarten aufgetragen und im Vergleich bewertet. Das +6°-Muster dient bei der Bewertung der Parfumprüfung als Standard. Entscheidend ist besonders die Bewertung bei 40°, da hier die Muster besonders gestresst werden.

### Beurteilung von Parfümprüfungen:

| **Note** | **Bewertung in Prozent [%]** | **Zuordnung** |
|---|---|---|
| 1 | 100 | 76 bis 100 % sehr gut |
| 1 - | 90 | |
| 2 + | 80 | |
| 2 | 70 | 51 bis 75 % gut / normal |
| 2 - | 60 | |
| 3 + | 50 | 26 bis 50 % akzeptabel |
| 3 | 40 | |
| 3 - | 30 | |
| 4 | 25 | 0 bis 25 % nicht akzeptabel |

### Ergebnisse:

| **Ansatz-Nr.** | **Lagerungsbedingung** | **nach 2 Monaten** | **nach 4 Monaten** | **nach 6 Monaten** |
|---|---|---|---|---|
| **1** | 6 °C | 100% | 100% | 100% |
| **1** | RT | 85% | 80% | 80% |
| **1** | 40 °C | 65% | 50% | 50% |
| **1** | Licht | 75% | 65% | 60% |
| **2** | 6 °C | 100% | 100% | 100% |
| **2** | RT | 95% | 90% | 85% |
| **2** | 40 °C | 85% | 85% | 80% |
| **2** | Licht | 80% | 85% | 90% |

### Rezepturvergleich:

| | **1** | **2** |
|---|---|---|
| **INCl** | **m [%]** | **m [%]** |
| Acrylates/C10-30 Alkyl Acrylat Crosspolymer | 0,1 | 0,1 |
| Butylen Glycol Dicaprylat/Dicaprat | 3,5 | 3,5 |
| Shea Butter | 3,0 | 3,0 |
| C12-15 Alkyl Benzoat | 1,0 | 1,0 |
| Caprylic/Capric Triglycerid | 1,0 | 1,0 |
| Cetearyl Alcohol | 4,0 | 4,0 |
| Cetyl Alcohol | 3,0 | 3,0 |
| Carraqeenan | 0,2 | 0,2 |
| Diethylamino Hydroxybenzoyl Hexyl Benzoat | 3,0 | 3,0 |
| Dimethicon | 0,5 | 0,5 |
| Ethylhexyl Salicylat | 3,8 | 3,8 |
| **Linalool, Hexylcinnamal, Benzylsalicylat** | | **0,02** |
| **Hexylcinnamal** | | **0,01** |
| **Benzylsalicylat** | | **0,02** |
| Parfum | 0,4 | 0,35 |
| Glycerin | 8,7 | 8,7 |
| Glyceryl Stearat SE | 2,6 | 2,6 |
| Methylparaben | 0,1 | 0,1 |
| Phenylbenzimidazol Sulfonsäure | 1,5 | 1,5 |
| Titandioxid | 0,8 | 0,8 |
| Tocopherol Acetat | 0,5 | 0,5 |
| EDTA | 1,0 | 1,0 |
| Wasser | ad 100,0 | ad 100,0 |
| **Summen:** | **100,0** | **100,0** |

### Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

### O/W Emulsion

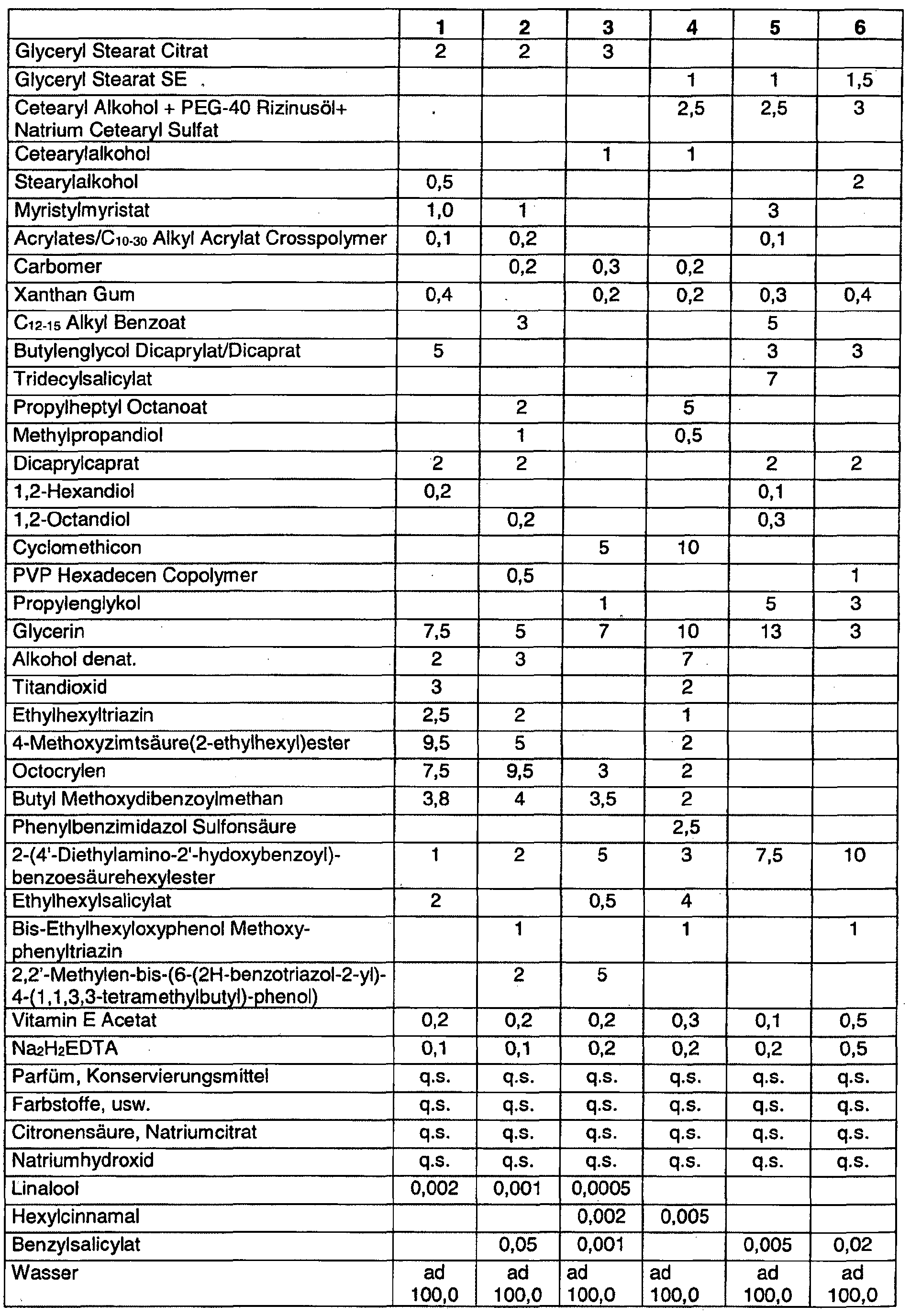

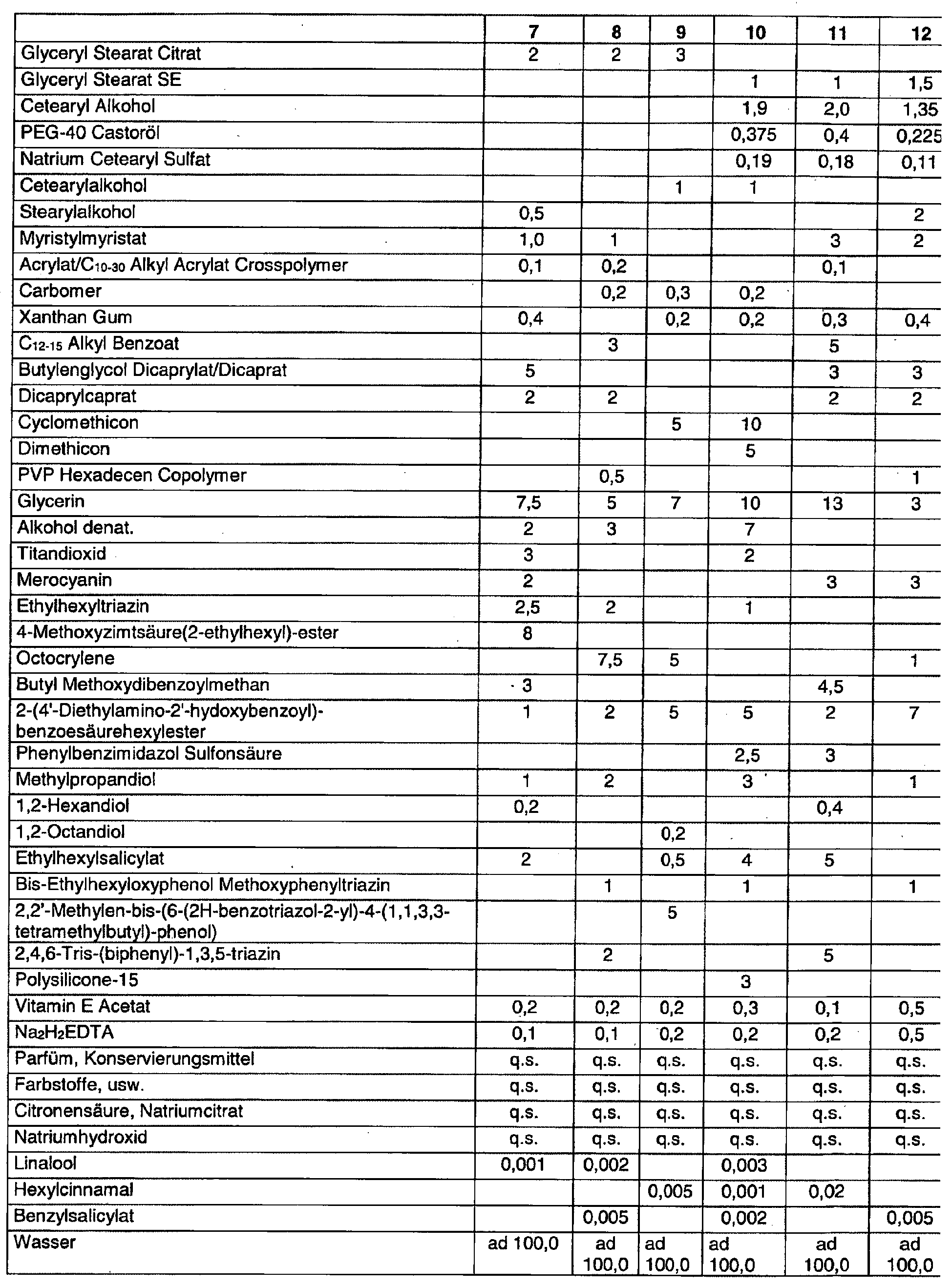

### Hydrodispersionen

| | **1** | **2** | **3** | **4** | **5** | **6** |
|---|---|---|---|---|---|---|
| Glyceryl Stearat Citrat | | 0,40 | | | | |
| Sodium Carbomer | | | | | 0,30 | |
| Copolymer aus Vinylpyrrolidon und Acrylsäure | 1,0 | 1,5 | | | | |
| Natrium Stearoyl Glutamate | 0,1 | | | | | |
| Acrylates/C10-30 Alkyl Acrylate Crosspolymer | | | 0,30 | 0,40 | 0,10 | 0,10 |
| Ceteareth-20 | | | 1,00 | | | |
| Xanthan Gummi | 0,50 | | | 0,15 | | 0,50 |
| Dimethicon / Vinyl Dimethicon Crosspolymer | | | | 5,00 | | 3,00 |
| 2-(4'-Diethylamino-2'-hydoxybenzoyl)-benzoesäurehexylester | 0,25 | 5,00 | 7,00 | 0,50 | 2,00 | 1,50 |
| Bis-Ethylhexyloxyphenol Methoxypheny Triazin | | 2,00 | | 0,25 | | |
| Ethylhexyl Methoxycinnamat | | | 7,00 | | | 8,00 |
| Diethylhexyl Butamido Triazin | | | 2,00 | 1,00 | | |
| Ethylhexyl Triazin | 4,00 | 3,00 | | | | |
| Titandioxid | 0,50 | | 1,00 | | | |
| Octocrylen | | 9,5 | 3 | 2 | | 7 |
| Butyl Methoxydibenzoylmethan | 4,2 | | 3,5 | | 4,5 | 1 |
| Methylpropandiol | 0,5 | 2 | 1,5 | 2,5 | 3 | 2 |
| C₁₂₋₁₅ Alkyl Benzoat | 2,00 | | 2,50 | | | |
| Ölsäure | 0,5 | 1 | 2 | 0,5 | 5 | 1 |
| C18-36 Triglycerid Fettsäure | | | 1,00 | | | |
| Butylenglycol Dicaprylat/Dicaprat | 4,00 | | | | 6,00 | |
| Dicaprylyl Carbonat | | 3,00 | | | | |
| Dicaprylylether | | 2,00 | | | | |
| Cyclomethicon | | | | 7,50 | | |
| PVP Hexadecen Copolymer | 0,50 | | 0,50 | | 0,50 | 1,00 |
| Glycerin | 10,00 | 5,00 | 5,00 | | 5,00 | 15,00 |
| Butylenglycol | | 7,00 | | | | |
| 1 ,2-Hexandiol | 0,50 | | | 1,00 | | 1,50 |
| 1,2-Octandiol | | 0,4 | 0,2 | | 0,1 | |
| Vitamin E Acetat | 0,50 | 0,25 | 0,50 | 0,25 | 0,5 | 1,00 |
| Panthenol | 1,50 | 0,50 | | | 0,25 | |
| Trinatrium EDTA | | 1,00 | 1,00 | 0,10 | 0,20 | |
| Ethanol | 3,00 | | 4,00 | 3,50 | | 1,00 |
| Konservierungsmittel | q.s. | q.s. | q.s. | q.s. | q.s. | q.s. |
| Linalool | 0,01 | | 0,001 | | 0,001 | |
| Hexylcinnamal | | 0,005 | 0,001 | 0,1 | | |
| Benzylsalicylat | | | 0,002 | | 0,005 | 0,001 |
| Parfüm, Farbstoffe, | q.s. | q.s. | q.s. | q.s. | q.s. | q.s. |
| Wasser | ad 100 | ad 100 | ad 100 | ad 100 | ad 100 | ad 100 |

### Gele

| | **1** | **2** | **3** | **4** | **5** | **6** |
|---|---|---|---|---|---|---|
| Acrylat/Octylacrylamid Copolymer | 1,0 | 1,0 | 1,0 | 1,0 | 1,0 | 1,0 |
| Alkohol Denat. | 50,0 | 62,0 | 59,2 | 70,0 | 70,0 | 69,0 |
| Butylen Glycol Dicaprylat/Dicaprat | | | 9,5 | | | |
| C12-15 Alkyl Benzoat | 5,0 | 10,0 | 5,0 | 5,0 | 9,5 | |
| Phenylbenzoat | 5,0 | | | | | |
| Cocoglycerid | | | | | | 5 |
| Diethylamino Hydroxybenzoyl Hexyl Benzoat | 1,5 | 1 | 4,5 | 3,5 | 2,5 | 5 |
| Ethylhexyl Methoxycinnamat | 5 | 4,5 | | | 0,5 | |
| Ethylhexyl Salicylat | 4,5 | | 4,5 | 4,5 | | |
| Homosalat | | | | | | 4,5 |
| Hydroxypropylcellulose | 2 | 0,8 | 1 | 0,8 | 0,5 | 0,8 |
| Octocrylen | 7,5 | 9,5 | 3 | 2 | 2 | 3 |
| Butyl Methoxydibenzoylmethan | 4,2 | 4 | 3,5 | 2 | 4,5 | 3 |
| Methylpropandiol | 0,5 | 2 | 1,5 | 2,5 | 3 | 2 |
| 1,2-Hexandiol | | 0,3 | | 0,2 | | |
| 1,2-Octandiol | 0,4 | | 0,1 | 0,2 | 0,5 | 0,1 |
| Ethylhexyltriazin | | 2 | | | 2 | |
| Benzophenone-3 | 3 | | | | | |
| Bis-Ethylhexyloxyphenol Methoxyphenyltriazin | | | 2,5 | | 3 | 1 |
| Ölsäure | 0,5 | 1 | 2 | 1,5 | 0,5 | 3 |
| Vitamin E Acetat | | | | 0,5 | | 0,2 |
| Glycerin | 5 | | 3 | | | |
| Parfüm, Farbstoffe | q.s. | q.s. | q.s. | q.s. | q.s. | q.s. |
| Llnalool | 0,001 | 0,002 | 0,005 | | | |
| Hexylcinnamal | | | 0,001 | 0,005 | | 0,001 |
| Benzylsalicylat | | | 0,002 | | 0,005 | |
| Wasser | ad 100 | ad 100 | ad 100 | ad 100 | ad 100 | ad 100 |

### Gesichtscreme

| **INCI** | **1** | **2** | **3** |
|---|---|---|---|
| Methylparaben | - | 0,1 | 0,2 |
| Glyceryl Stearat | - | 3 | - |
| Caprylic/Capric Triglycerid | 1 | 1,5 | - |
| Mikrokristallines Wachs | 0,7 | 1,5 | - |
| Parafinöl | 0,5 | 1 | - |
| Vitamin E Acetat | 0,5 | 0,5 | - |
| Cetearyl Alkohol | 4 | 4 | 1,5 |
| Cetyl Alkohol | 3 | - | 1,5 |
| Glyceryl Stearat SE | 2,6 | 2,6 | - |
| Trisodium EDTA | 1 | 1 | 1 |
| BHT | - | - | 0,01 |
| Ethylhexyl Methoxycinnamat | - | - | 7,5 |
| Phenoxyethanol | - | 0,4 | 0,4 |
| Dimethicon | 0,5 | 0,5 | 2 |
| Hydrogenierte Coco-Glyceride | 2 | - | 4 |
| C12-15 Alkyl Benzoat | 1 | 2 | - |
| Butyl Methoxydibenzoylmethan | - | 3 | 2 |
| Phenylbenzimidazol Sulfonsäure | 1,5 | 1,4 | - |
| Glycerin | 8,7 | 8,7 | 9,7 |
| Natronlauge | pH-Einstellung | pH-Einstellung | pH-Einstellung |
| Butylene Glycol | - | - | 3 |
| Ethylhexyl Salicylate | 3,75 | - | 4,5 |
| Karietébutter | 3 | 3 | 2,5 |
| Ölsäure | 0,5 | 1 | 0,2 |
| Acrylats/C10-30 Alkyl Acrylat Crosspolymer | 0,1 | 0,1 | 0,1 |
| Titan Dioxid + Trimethoxycaprylylsilan | - | 0,5 | - |
| Glyceryl Stearat Citrat | - | - | 2 |
| Cl 15985 | - | 0,7 | 0,5 |
| Cl 16035 | 0,5 | 0,2 | - |
| Ethylhexylglycerin | 0,25 | 0,5 | 0,25 |
| Octocrylene | - | 3 | - |
| Pentylen Glycol | 1 | - | - |
| Chondrus Crispus | 0,2 | 0,2 | - |
| Bis-Ethylhexyloxyphenol Methoxyphenyl Triazin | - | 1,75 | 0,5 |
| Butylen Glycol Dicaprylat/Dicaprat | 3,5 | - | - |
| Methylpropandiol | 2 | 4 | 3 |
| Diethylamino Hydroxybenzoyl Hexyl Benzoate | 3 | 2 | 4,5 |
| 1,2-Hexandiol | 1 | - | - |
| Titan Dioxid + Aluminum Hydroxid + Dimethicon/Methicon Copolymer | 0,75 | - | 1 |
| Sodium Hyaluronat | 0,1 | 1 | 0,5 |
| Aqua + Pimpinella Anisum Extract | 5 | 2,5 | 4 |
| Parfum | 0,3 | 0,4 | 0,2 |
| Linalool | 0,05 | 0,06 | 0,03 |
| Hexylcinnamal | 0,03 | 0,04 | 0,01 |
| Benzylsalicylat | 0,01 | 0,03 | 0,02 |
| Wasser | ergänze zu 100 | ergänze zu 100 | ergänze zu 100 |

## Patentansprüche

1. Kosmetische Zubereitung in Form einer Emulsion oder Dispersion enthaltend
a) 2-(4'-Diethylamino-2'-hydoxybenzoyl)-benzoesäurehexylester sowie
b) eine oder mehrere Parfümstoffe gewählt aus der Gruppe der Verbindungen Linalool, Hexylcinnamal, Benzylsalicylat und
c) einen oder mehrere weitere UV-Filter gewählt aus der Gruppe der Verbindungen Phenylen-1,4-bis-(2-benzimidazyl)-3,3'-5,5'-tetrasulfonsäuresalze; 2-Phenylbenzimidazol-5-sulfonsäuresalze; 1,4-di(2-oxo-10-Sulfo-3-bornylidenmethyl)-Benzol und dessen Salze; 4-(2-Oxo-3-bornylidenmethyl)benzolsulfonsäuresalze; 2-Methyl-5-(2-oxo-3-bornylidenmethyl)-sulfonsäuresalze; 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; Ethylhexylsalicylat; Terephthalidendicamphersulfonsäure; 4-(Dimethylamino)-benzoesäure(2-ethylhexyl)ester; 4-(Dimethylamino)benzoesäure-amylester; 4-Methoxybenzalmalon-säuredi(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; 4-(tert.-Butyl)-4'-methoxydibenzoylmethan; Homomenthylsalicylat; 2-Ethylhexyl-2-hydroxybenzoat; 2-Ethylhexyl-2-cyano-3,3-diphenylacrylat; Dimethicodiethylbenzalmalonat; 3-(4-(2,2-bis Ethoxycarbonylvinyl)-phenoxy)propenyl)-methoxysiloxan / Dimethylsiloxan - Copolymer; Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin); 2,4,6-Tris-(biphenyl)-1,3,5-triazin; 2,4-Bis-(4'-Di-neopentylaminobenzalmalonat)-6-(4"-butylaminobenzoat)-s-triazin ; Titandioxid, Zinkoxid, Merocyanine gewählt aus der Gruppe der Verbindungen 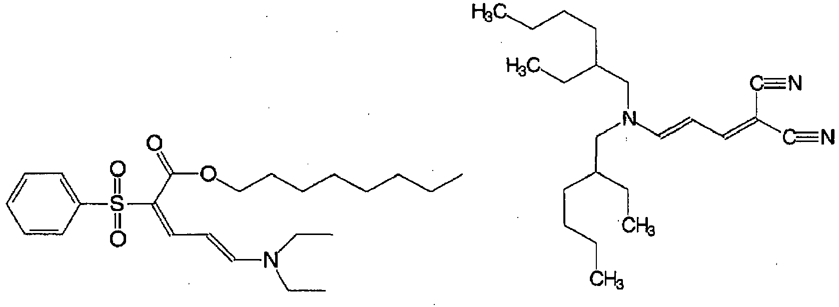 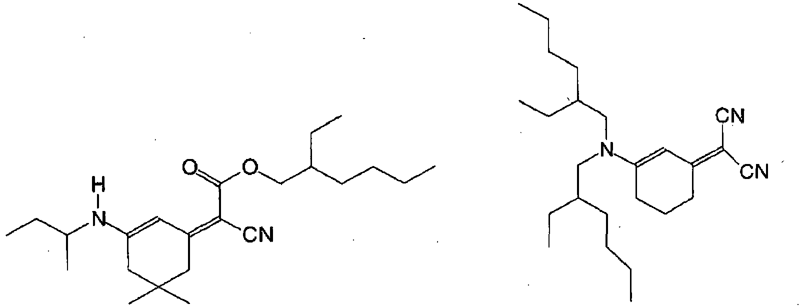 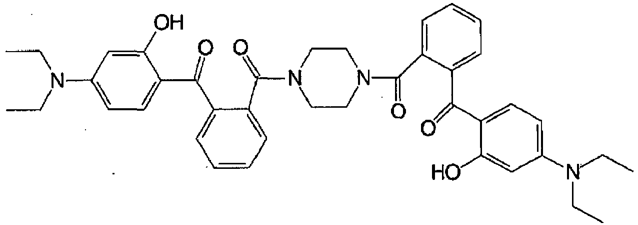 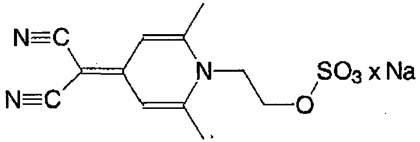 wobei die Gesamtkonzentration an Parfümstoffen in der Zubereitung von 0,00001 bis 1%, bezogen auf das Gesamtgewicht der Zubereitung, beträgt.

2. Kosmetische Zubereitung nach Anspruch 1, **dadurch gekennzeichnet, dass** die Zubereitung 2-(4'-Diethylamino-2'-hydoxybenzoyl)-benzoesäurehexylester in einer Konzentration von 0,1 bis 10 Gew.-%, bezogen auf das Gesamtgewicht der Zubereitung, beträgt.

3. Kosmetische Zubereitung nach einem der vorhergehenden Ansprüche, **dadurch gekennzeichnet, dass** die Zubereitung als weitere Komponenten eine oder mehrere Verbindungen gewählt aus der Gruppe der Verbindungen Limonen, Citral, alpha-Isomethylionon, Geraniol, Methyl-2-Octynoat, Citronellol, Isoeugenol, 2-Isobutyl-4-hydroxy-4-methyltetrahydropyran, 2-tert-Pentylcyclohexylacetat, 3-Methyl-5-phenyl-1-pentanol, 7-Acetyl-1,1,3,4,4,6-hexamethyltetralin, Adipinsäurediester, alpha-Amylcinnamaldehyd, Alpha-Methylionon, Amyl C Butylphenylmethylpropionalcinnamal, Amylsalicylat, Amylcinnamylalkohol, Anisalkohol, Benzoin, Benzylalkohol, Benzylbenzoat, Benzylcinnamat, Bergamotöl, bitteres Orangenöl, Butylphenylmethylpropioal, Cardamomöl, Cedrol, Cinnamal, Cinnamylalkohol, Citronellylmethylcrotonat, Citronenöl, Coumarin, Diethylsuccinat, d-Limonene, Ethyllinalool, Eugenol, Evernia Furfuracea Extract, Evernia Prunastri Extract, Farnesol, Guajakholzöl, Hexylsalicylat, Hydroxycitronellal, Hydroxyisohexyl 3-Cyclohexencarboxaldehyde, Lavendelöl, Lemonenöl, Linaylacetat, Mandarinenöl, Menthyl PCA, Methylheptenon, Muskatnussöl, Rosmarinöl, süßes Orangenöl, Terpineol, Tonkabohnenöl, Triethylcitrat und/oder Vanillin.

4. Kosmetische Zubereitung nach einem der vorhergehenden Ansprüche, **dadurch gekennzeichnet, dass** die Zubereitung als weitere Inhaltsstoffe eine oder mehrere Verbindungen gewählt aus der Gruppe der Verbindungen alpha-Liponsäure, Folsäure, Phytoen, D-Biotin, Coenzym Q10, alpha-Glucosylrutin, Carnitin, Carnosin, natürliche und/oder synthetische Isoflavonoide, Flavonoide, Kreatin, Kreatinin, Taurin, ß-Alanin, Tocopherylacetat, Dihydroxyaceton; 8-Hexadecen-1,16-dicarbonsäure, Glycerylglycose, (2-Hydroxyethyl)harnstoff, Niacinamid, Vitamin E bzw. seine Derivate und/oder Licochalcon A enthält.

5. Kosmetische Zubereitung nach einem der vorhergehenden Ansprüche, **dadurch gekennzeichnet, dass** die Zubereitung ein oder mehrere Diole gewählt aus der Gruppe der Verbindungen 2-Methyl-1,3-propandiol, Pentan-1,2-diol, Hexan-1,2-diol, Heptan-1,2-diol, Octan-1,2-diol, Nonan-1,2-diol, Decan-1,2-diol enthält.

6. Kosmetische Zubereitung nach einem der vorhergehenden Ansprüche, **dadurch gekennzeichnet, dass** die Zubereitung ein oder mehrere Parabene enthält.

## Claims

1. Cosmetic preparation in the form of an emulsion or dispersion comprising
a) hexyl 2-(4'-diethylamino-2'-hydroxybenzoyl)benzoate and
b) one or more perfume substances selected from the group of compounds comprising linalool, hexylcinnamal, benzyl salicylate and
c) one or more further UV filters selected from the group of compounds comprising phenylene-1-4-bis(2-benzimidazyl)-3,3'-5,5'-tetrasulfonic acid salts; 2-phenylbenzimidazole-5-sulfonic acid salts; 1,4-di(2-oxo-10-sulfo-3-bornylidenemethyl)benzene and salts thereof; 4-(2-oxo-3-bornylidenemethyl)benzenesulfonic acid salts; 2-methyl-5-(2-oxo-3-bornylidenemethyl)sulfonic acid salts; 2-(2H-benzotriazol-2-yl)-4-methyl-6-[2-methyl-3-[1,3,3,3-tetramethyl-1-[(trimethylsilyl)oxy]disiloxanyl]propyl]phenol; 3-(4-methylbenzylidene)camphor; 3-benzylidenecamphor; ethylhexyl salicylate; terephthalidenedicamphorsulfonic acid; 2-ethylhexyl 4-(dimethylamino)benzoate; amyl 4-(dimethylamino)benzoate; di(2-ethylhexyl) 4-methoxybenzalmalonate; isoamyl 4-methoxycinnamate; 2-hydroxy-4-methoxybenzophenone, 2-hydroxy-4-methoxy-4'-methylbenzophenone; 2,2'-dihydroxy-4-methoxybenzophenone; 4-(tert-butyl)-4'-methoxydibenzoylmethane; homomenthyl salicylate; 2-ethylhexyl 2-hydroxybenzoate; 2-ethylhexyl 2-cyano-3,3-diphenylacrylate; dimethicodiethyl benzalmalonate; 3-(4-(2,2-bisethoxycarbonylvinyl)phenoxy)propenyl)methoxysiloxane/dimethylsiloxane copolymer; dioctylbutylamidotriazone (INCI: Diethylhexyl Butamidotriazone); 2,4-bis[5-1(dimethylpropyl)benzoxazol-2-yl-(4-phenyl)imino]-6-(2-ethylhexyl)imino-1,3,5-triazine with the CAS No. 288254-16-0; 2,4-bis{[4-(2-ethylhexyloxy)-2-hydroxy]phenyl}-6-(4-methoxyphenyl)-1,3,5-triazine (INCI: Bis-Ethylhexyloxyphenol Methoxyphenyl Triazine); 2,4,6-tris(biphenyl)-1,3,5-triazine; 2,4-bis(4'-dineopentylaminobenzalmalonate)-6-(4"-butylaminobenzoate)-s-triazine; titanium dioxide, zinc oxide, merocyanines selected from the group of compounds comprising 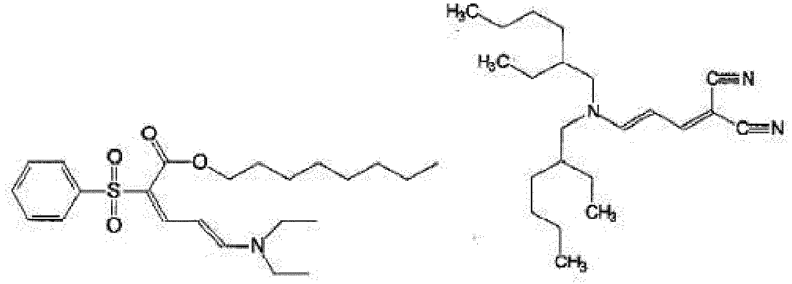 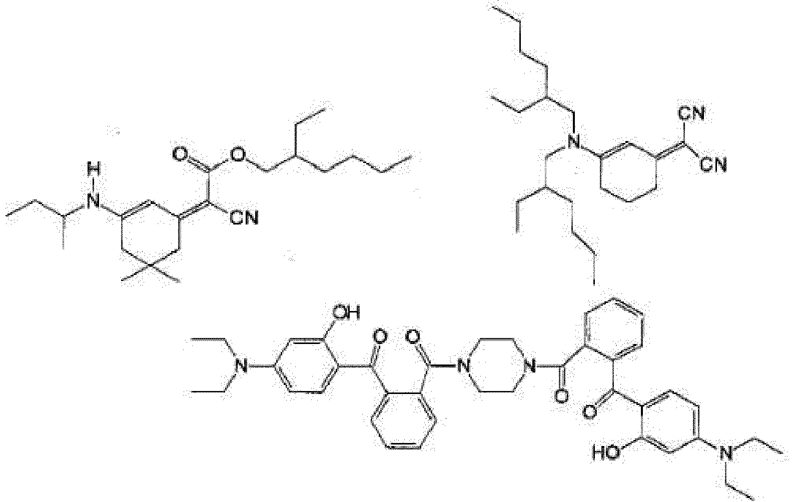 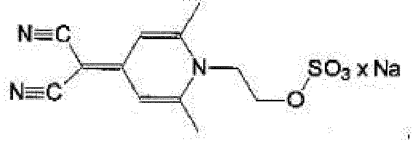 wherein the total concentration of perfume substances in the preparation is from 0.00001 % to 1%, based on the total weight of the preparation.

2. Cosmetic preparation according to Claim 1, **characterized in that** the preparation is hexyl 2-(4'-diethylamino-2'-hydroxybenzoyl)benzoate in a concentration of from 0.1 to 10% by weight, based on the total weight of the preparation.

3. Cosmetic preparation according to either of the preceding claims, **characterized in that** the preparation, as further components, one or more compounds selected from the group of compounds comprising limonene, citral, alpha-isomethylionone, geraniol, methyl 2-octynoate, citronellol, isoeugenol, 2-isobutyl-4-hydroxy-4-methyltetrahydropyran, 2-tert-pentyl cyclohexylacetate, 3-methyl-5-phenyl-1-pentanol, 7-acetyl-1,1,3,4,4,6-hexamethyltetralin, adipic acid diester, alpha-amylcinnamaldehyde, alpha-methylionone, amyl C, butylphenylmethylpropionalcinnamal, amyl salicylate, amylcinnamyl alcohol, anise alcohol, benzoin, benzyl alcohol, benzyl benzoate, benzyl cinnamate, bergamot oil, bitter orange oil, butylphenylmethylpropioal, cardamom oil, cedrol, cinnamal, cinnamyl alcohol, citronellyl methylcrotonate, lemon oil, coumarin, diethyl succinate, d-limonene, ethyllinalool, eugenol, Evernia furfuracea extract, Evernia prunastri extract, farnesol, guaiac wood oil, hexyl salicylate, hydroxycitronellal, hydroxyisohexyl 3-cyclohexenecarboxaldehyde, lavender oil, limonene oil, linalyl acetate, mandarin oil, menthyl PCA, methylheptenone, nutmeg oil, rosemary oil, sweet orange oil, terpineol, tonka bean oil, triethyl citrate and/or vanillin.

4. Cosmetic preparation according to any of the preceding claims, **characterized in that** the preparation comprises, as further ingredients, one or more compounds selected from the group of compounds comprising alpha-lipoic acid, folic acid, phytoene, D-biotin, coenzyme Q10, alpha-glucosylrutin, carnitine, carnosine, natural and/or synthetic isoflavonoids, flavonoids, creatine, creatinine, taurine, β-alanine, tocopheryl acetate, dihydroxyacetone; 8-hexadecene-1,16-dicarboxylic acid, glycerylglycose, (2-hydroxyethyl)urea, niacinamide, vitamin E and derivatives thereof and/or licochalcone A.

5. Cosmetic preparation according to any of the preceding claims, **characterized in that** the preparation comprises one or more diols selected from the group of compounds comprising 2-methyl-1,3-propanediol, pentane-1,2-diol, hexane-1,2-diol, heptane-1,2-diol, octane-1,2-diol, nonane-1,2-diol, decane-1,2-diol.

6. Cosmetic preparation according to any of the preceding claims, **characterized in that** the preparation comprises one or more parabens.

## Revendications

1. Préparation cosmétique sous forme d'une émulsion ou dispersion contenant
a) l'ester hexylique de l'acide 2-(4'-diéthylamino-2'-hydroxybenzoyl)-benzoïque ainsi que
b) une ou plusieurs substances parfumées choisies dans le groupe de composés linalool, hexylcinnamal, salicylate de benzyle et
c) un ou plusieurs autres filtres UV choisis dans le groupe des composés : sels de l'acide phénylène-1,4-bis-(2-benzimidazyl)-3,3'-5,5'-tétrasulfonique ; sels de l'acide 2-phénylbenzimidazole-5-sulfonique ; 1,4-di(2-oxo-10-sulfo-3-bornylidèneméthyl)-benzène et ses sels ; sels de l'acide 4-(2-oxo-3-bornylidèneméthyl)benzènesulfonique ; sels de l'acide 2-méthyl-5-(2-oxo-3-bornylidèneméthyl)sulfonique ; 2-(2H-benzotriazol-2-yl)-4-méthyl-6-[2-méthyl-3-(1,3,3,3-tétraméthyl-1-[(triméthylsilyl)oxy]disiloxanyl]propyl]-phénol ; 3-(4-méthylbenzylidène)camphre ; 3-benzylidènecamphre ; salicylate d'éthylhexyle ; acide téréphtalidènedicamphresulfonique ; ester 2-éthylhexylique de l'acide 4-(diméthylamino)-benzoïque ; ester amylique de l'acide 4-(diméthylamino)benzoïque ; ester di(2-éthylhexylique) de l'acide 4-méthoxybenzalmalonique ; ester isoamylique de l'acide 4-méthoxycinnamique ; 2-hydroxy-4-méthoxybenzophénone, 2-hydroxy-4-méthoxy-4'-méthylbenzophénone ; 2,2'-dihydroxy-4-méthoxybenzophénone ; 4-(tert-butyl)-4'-méthoxydibenzoylméthane ; salicylate d'homomenthyle ; 2-hydroxybenzoate de 2-éthylhexyle ; 2-cyano-3,3-diphénylacrylate de 2-éthylhexyle ; benzalmalonate de diméthicodiéthyle ; copolymère de 3-(4-(2,2-bis(éthoxycarbonylvinyl)-phénoxy)propényl)-méthoxysiloxane/diméthylsiloxane ; dioctylbutylamidotriazone (INCI : Diethylhexyl-Butamidotriazone) ; 2,4-bis-[5-1(diméthylpropyl)benzoxazol-2-yl-(4-phényl)-imino]-6-(2-éthylhexyl)-imino-1,3,5-triazine présentant le n° CAS 288254-16-0 ; 2,4-bis-{[4-(2-éthylhexyloxy)-2-hydroxy]-phényl}-6-(4-méthoxyphényl)-1,3,5-triazine (INCI : Bis-Ethylhexyloxyphenol Methoxyphenyl Triazin) ; 2,4,6-tris-(biphényl)-1,3,5-triazine ; 2,4-bis-(4'-aminobenzalmalonate de dinéopentyle)-6-(4"-aminobenzoate de butyle)-s-triazine ; dioxyde de titane, oxyde de zinc, mérocyanines choisies dans le groupe des composés 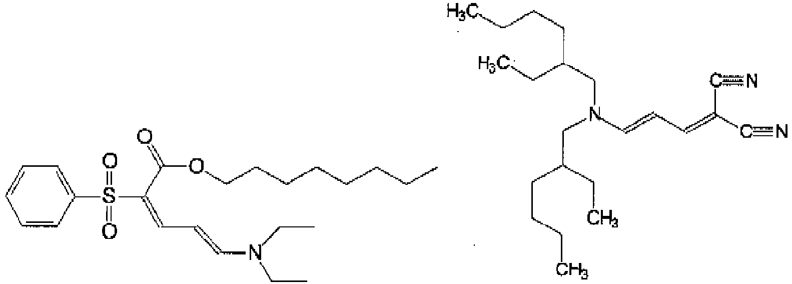 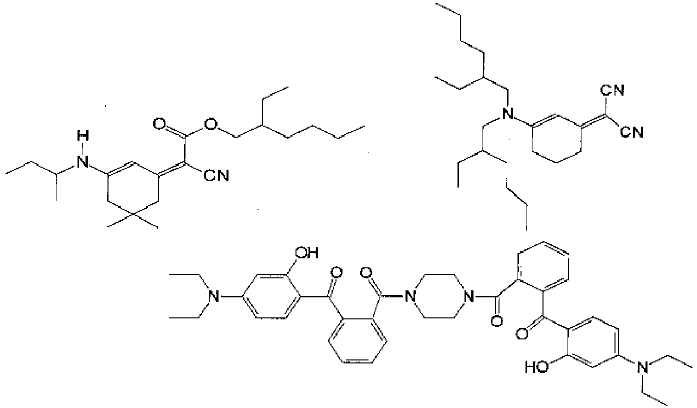 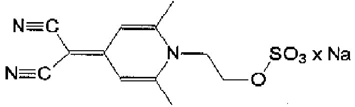 la concentration totale en substances parfumées dans la préparation étant de 0,00001 à 1 %, par rapport au poids total de la préparation.

2. Préparation cosmétique selon la revendication 1, **caractérisée en ce que** la préparation est l'ester hexylique de l'acide 2-(4'-diéthylamino-2'-hydroxybenzoyl)-benzoïque en une concentration de 0,1 à 10 % en poids, par rapport au poids total de la composition.

3. Préparation cosmétique selon l'une quelconque des revendications précédentes, **caractérisée en ce que** la composition, comme autres composants, un ou plusieurs composés choisis dans le groupe des composés limonène, citral, alpha-isométhylionone, géraniol, 2-octynoate de méthyle, citronellol, iso-eugénol, 2-isobutyl-4-hydroxy-4-méthyltétrahydropyrane, acétate de 2-tert-pentylcyclohexyle, 3-méthyl-5-phényl-1-pentanol, 7-acétyl-1,1,3,4,4,6-hexaméthyltétraline, diester de l'acide adipique, alpha-amylcinnamaldéhyde, alpha-méthylionone, Amyl C, butylphénylméthylpropionalcinnamal, salicylate d'amyle, alcool amylcinnamylique, alcool anisique, benzoïne, alcool benzylique, benzoate de benzyle, cinnamate de benzyle, huile de bergamote, huile d'orange amère, butylphénylméthylpropioal, huile de cardamome, cédrol, cinnamal, alcool cinnamylique, crotonate de citronellylméthyle, huile de citron, coumarine, succinate de diéthyle, d-limonène, éthyllinalool, eugénol, extrait d'Evernia Furfuracea, extrait d'Evernia Prunastri, farnésol, huile de bois de gaïac, salicylate d'hexyle, hydroxycitronellal, hydroxyisohexyl-3-cyclohexènecarboxaldéhyde, huile de lavande, huile de limonène, acétate de linalyle, huile de mandarine, menthyl-PCA, méthylhepténone, huile de noix de muscade, huile de romarin, huile d'orange douce, terpinéol, huile de fèves de tonka, citrate de triéthyle et/ou vanilline.

4. Préparation cosmétique selon l'une quelconque des revendications précédentes, **caractérisée en ce que** la préparation contient, comme autres constituants, un ou plusieurs composés choisis dans le groupe des composés acide alpha-lipoïque, acide folique, phytoène, D-biotine, coenzyme Q10, alpha-glucosylrutine, carnitine, carnosine, isoflavonoïdes naturels et/ou synthétiques, flavonoïdes, créatine, créatinine, taurine, β-alanine, acétate de tocophéryle, dihydroxyacétone ; acide 8-hexadécène-1,16-dicarboxylique, glycérylglucose, (2-hydroxyéthyl)urée, niacinamide, vitamine E ou, selon le cas, ses dérivés et/ou licochalcone A.

5. Préparation cosmétique selon l'une quelconque des revendications précédentes, **caractérisée en ce que** la préparation contient un ou plusieurs diols choisis dans le groupe des composés 2-méthyl-1,3-propanediol, pentane-1,2-diol, hexane-1,2-diol, heptane-1,2-diol, octane-1,2-diol, nonane-1,2-diol, décane-1,2-diol.

6. Préparation cosmétique selon l'une quelconque des revendications précédentes, **caractérisée en ce que** la préparation contient un ou plusieurs parabènes.
